# EUROPÄISCHE PATENTSCHRIFT

(11) **EP 0 671 418 B1**
(45) Veröffentlichungstag und Bekanntmachung des Hinweises auf die Patenterteilung: **12.08.1998**
(21) Anmeldenummer: 95101664.1
(22) Anmeldetag: 08.02.1995
(51) Int. Cl.: C08F 8/30, A61L 15/60, C08J 3/24

(54) **Hydrophile, hochquellfähige Hydrogele**
Hydrophilic hydrogels having a high swelling capacity
Hydrogels hydrophiles à forte capacité de gonflement

(30) Priorität: 12.03.1994 DE 4408435
(43) Veröffentlichungstag der Anmeldung: 13.09.1995
(73) Patentinhaber: Clariant GmbH, 65929 Frankfurt am Main (DE)
(72) Erfinder: Engelhardt, Friedrich, Dr., D-60386 Frankfurt am Main (DE); Riegel, Ulrich, D-60386 Frankfurt am Main (DE); Stüven, Uwe, D-65812 Bad Soden (DE); Klotzsche, Helmut, D-63755 Alzenau (DE); Remmel, Gustav, D-63571 Gelnhausen (DE)

(56) Entgegenhaltungen:
- EP-A- 0 159 371
- EP-A- 0 493 011
- EP-A- 0 618 005
- DE-C- 2 614 662

## Beschreibung

Die vorliegende Erfindung betrifft hydrophile, hochquellfähige Hydrogele, die mit Polyamidoaminen in wäßrigem Medium nachvernetzt sind.

Hydrophile Hydrogele, die durch Polymerisation olefinisch ungesättigter Säuren, wie beispielsweise Acrylsäure, Methacrylsäure, Acrylamidopropansulfonsäure usw., in Gegenwart geringer Mengen mehrfach olefinisch ungesättigter Verbindungen erhalten werden können, sind bereits bekannt und beispielsweise beschrieben in US 4,057,521, US 4,062,817, US 4,525,527, US 4,286,082, US 4,340,706 und US 4,295,987.

Weiterhin sind auch hydrophile Hydrogele bekannt, die durch Pfropfcopolymerisation olefinisch ungesättigter Säuren auf unterschiedliche Matrices, wie beispielsweise Polysaccharide, Polyalkylenoxide sowie deren Derivate, zugänglich sind (z.B. US 5,011,892, US 4,076,663 und US 4,931,497).

Die genannten Hydrogele zeichnen sich durch ein hohes Aufnahmevermögen für Wasser und wäßrige Lösungen aus und finden daher bevorzugt Anwendung als Absorptionsmittel in Hygieneartikeln.

Es ist bereits bekannt, daß die Eigenschaften dieser Hydrogele durch eine Oberflächenbehandlung mit bestimmten Substanzen modifiziert werden können. Zu diesem Zweck werden herkömmliche Hydrogele, die getrocknet, gemahlen und gegebenenfalls abgesiebt sind, in Pulverform mit Verbindungen umgesetzt, die mindestens zwei Gruppen enthalten, die mit den Carboxylgruppen der Hydrogele kovalente Bindungen bilden können. Diese Vernetzung findet nur auf der Oberfläche der Gelpartikel statt, nicht aber in ihrem Innern.

Eine derartige Oberflächenvernetzung ist beispielsweise in der EP-A 543 303 beschrieben, wobei als Oberflächenvernetzer Mischungen aus Phosphonsäurediglycidylestern und weiteren reaktiven Verbindungen, z.B. Polyamidoaminen, eingesetzt werden.

Es ist auch bereits bekannt, Hydrogele unmittelbar nach Abschluß der Polymerisationsreaktion und gegebenenfalls (Teil)Neutralisierung der Carboxylgruppen mit Lauge in wässriger Gelphase nachzuvernetzen. Als dazu geeignete Vernetzer nennt die EP-A 530 438 beispielsweise Ethylenglykoldiglycidylether, Epichlorhydrin und Ethylendiamin.

DE-A-26 14 662 offenbart ein Verfahren zur Herstellung von in Wasser quellbaren Hydrogelen, insbesondere die Vernetzung von nicht vorvernetzten, wasserlöslichen carboxylischen Acrylesterpolymerisaten mit Polyamido/Polyamin/Epichlorhydrin-Addukten. Eine zusätzliche Vernetzung auf der Oberfläche und im Teilcheninnern wird nicht offenbart.

EP-A-0 159 371 offenbart ein absorbierendes Material, das einen leicht vernetzten Polyelekrolyten auf der Basis von Alkalicarboxylaten enthält. Die Vernetzung erfolgt mit Polyamido/Polyamin/Epichlorhydrin-Addukten. Eine zusätzliche Vernetzung auf der Oberfläche und im Teilcheninnern wird auch hier nicht offenbart.

EP-A-0 493 011 offenbart ein wasserabsorbierendes Material, das durch die Behandlung eines wasserabsorbierenden Partikels mit einem Vernetzer entsteht. Es wirs ausdrücklich eine Vernetzung auf der Oberfläche offenbart, keine Vernetzung im Teilcheninnern.

Es wurde nun gefunden, daß Hydrogele mit hervorragenden Produkteigenschaften erhalten werden, wenn sie mit Polyamidoaminen nachvernetzt werden.

Die vorliegende Erfindung betrifft somit ein hydrophiles, hochquellfähiges Hydrogel auf Basis vorvernetzter (co)polymerisierter hydrophiler Monomerer oder auf Basis vorvernetzter Pfropf(co)polymerer, dadurch gekennzeichnet, daß es mit einem Polyamidoamin in wäßrigem Medium nachvernetzt ist.

Polyamidoamine sind Verbindungen, die durch Umsetzung einer Säurekomponente wie zum Beispiel einer Dicarbonsäure oder eines funktionellen Derivates davon, oder einer ω-Aminocarbonsäure oder deren Lactam mit einer Aminkomponente wie zum Beispiel eines Polyamins, das auch im Gemisch mit Alkanolmonoaminen vorliegen kann, erhalten werden können und die partiell mit Epichlorhydrin quarternisiert sein können.

Geeignete Polyamine enthalten mindestens zwei primäre Aminogruppen und vorzugsweise mindestens eine sekundäre und/oder tertiäre Aminogruppe. Das Mengenverhältnis zwischen Säure- und Aminokomponente wird dabei vorzugsweise so gewählt, daß im resultierenden Polyamidoamin noch eine genügende Zahl an basischen Aminogruppen vorhanden ist, die mit Epichlorhydrin quarternisierbar sind. Polyamidoamine der genannten Art sind bekannt und beispielsweise in DE-A 17 77 824, GB-B 865.727, US 4.075.177, US 4.336,835, DE-A 33 23 732, EP-A 31 899 und EP-A 512 423 beschrieben und können nach den dort angegebenen Verfahren hergestellt werden.

Geeignete Polyamidoamine sind insbesondere Umsetzungsprodukte von
a) gesättigten oder olefinisch-ungesättigten aliphatischen C₃-C₁₀-Dicarbonsäuren oder deren funktionellen Derivaten bzw. mindestens 3 C-Atome enthaltenden ω-Aminocarbonsäuren oder deren Lactamen mit
b1) aliphatischen Polyaminen, die mindestens zwei primäre und mindestens eine weitere, und zwar sekundäre und/oder tertiäre Aminogruppe enthalten, oder
b2) einem Gemisch der Polyamine b1) mit
c1) Polyaminen, die mindestens eine primäre und gegebenenfalls mindestens eine sekundäre Aminogruppe enthalten und nicht unter die Definition b1) fallen, wobei diese Polyamine in solchen Mengen verwendet werden können, daß deren Anteil an primären Aminogruppen, bezogen auf die Gesamtmenge der primären Aminogruppen, 70 %, vorzugsweise 50 % und insbesondere 30 %,nicht übersteigt, und/oder gegebenenfalls
c2)Alkanolmonoaminen mit 2 bis 20 C-Atomen, vorzugsweise 2 bis 6 C-Atomen und 1 bis 3, vorzugsweise 1 oder 2 OH-Gruppen und insbesondere mit einer OH-Gruppe. Vorzugsweise besitzen diese Alkanolmonoamine die weiter unten angegebene Formel (II).

Als bevorzugte Polyamidoamine kommen in Betracht:
1. Umsetzungsprodukte von a) gesättigten aliphatischen C₃-C₁₀-Dicarbonsäuren, wie Bernsteinsäure, (C₁-C₆)-Alkyl- bzw. (C₁-C₆)-Alkylenbernsteinsäure, Glutarsäure, Adipinsäure, Sebazinsäure, Malonsäure oder deren funktionellen Derivaten, wie Anhydriden und Estern, wobei die drei erstgenannten Säuren bevorzugt sind, mit den vorstehend unter b) genannten Gemischen von Polyaminen mit Alkanolmonoaminen.
2. Umsetzungsprodukte von solchen gesättigten modifizierten Dicarbonsäuren bzw. deren Derivaten, die durch Anlagerung von aliphatischen, cycloaliphatischen, araliphatischen oder heterocyclischen Polyaminen, die mindestens zwei Aminogruppen und zwar primäre und/oder sekundäre enthalten, an α,β-olefinisch-ungesättigte Carbonsäureester, deren Alkoholrest 1 bis 8, vorzugsweise 1 bis 3 C-Atomen hat, wie Acrylsäureethylester und Methacrylsäuremethylester, erhalten und dann mit den vorstehend unter b₁)/c₁) genannten Polyaminen bzw. deren Gemischen, gegebenenfalls in Abmischung mit Alkanolmonoaminen, umgesetzt werden.
3. Umsetzungsprodukte von mindestens drei Kohlenstoffatomen enthaltenden ω-Aminocarbonsäuren oder deren Lactamen, z.B. 6-Aminocapronsäure und 8-Aminocaprylsäure bzw. 6-Caprolactam und 8-Capryllactam, mit den vorstehend unter b) genannten Gemischen von Polyaminen mit Alkanolmonoaminen.
4. Umsetzungsprodukte von olefinisch ungesättigten Dicarbonsäuren, wie Maleinsäure oder Fumarsäure bzw. deren funktionellen Derivaten, wie Anhydriden oder Estern, mit den vorstehend unter b₁)/c₁) genannten Polyaminen bzw. deren Gemischen, gegebenenfalls in Abmischung mit Alkanolmonoaminen.
5. Solche Umsetzungsprodukte, denen außer den unter 1. genannten gesättigten, aliphatischen C₃-C₁₀-Dicarbonsäuren und den unter 4. genannten ungesättigten Dicarbonsäuren noch ω-Aminocarbonsäuren oder deren Lactame des unter 3. angeführten Typs und außerdem unter b1) angeführten Polyaminen noch mindestens eine primäre und gegebenenfalls sekundäre Aminogruppe, also z.B. eine primäre oder eine sekundäre Aminogruppe, enthaltende aliphatische, cycloaliphatische, araliphatische oder heterocyclische Polyamine und Alkanolamine zugrunde liegen. Hierbei sind diejenigen Polyamidoamine zu bevorzugen, denen außer den vorstehend unter b₁)/c₁) genannten Polyaminen bzw. deren Gemischen, gegebenenfalls in Abmischung mit Alkanolmonoaminen und außer den gesättigten C₃-C₁₀-Dicarbonsäuren noch ω-Aminocarbonsäuren oder deren Lactame des unter 3. angeführten Typs zugrunde liegen.

Umsetzungsprodukte vom Typ 1) unter Verwendung von Ethylendiamin, Diethylentriamin, Triethylentetramin und Tetraethylenpentamin als Polyamin und 2-Aminoethanol als Alkanolmonoamin sind besonders bevorzugt.

Als Polyamine b1) und c1) kommen beispielsweise solche der Formel (I) 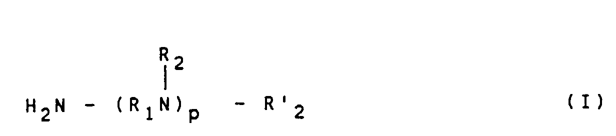 in Betracht, in der
pNull oder eine ganze Zahl von 1 bis 6, vorzugsweise 1 bis 4 ist,
R₁ einen zweiwertigen, vorzugsweise nichtaromatischen Kohlenwasserstoffrest mit 2 bis 18 C-Atomen, vorzugsweise einen verzweigten oder unverzweigten Alkylenrest mit 2 bis 10 C-Atomen, insbesondere mit 2 bis 6 C-Atomen, oder einen Cycloalkylenrest mit 5 bis 12 C-Atomen, vorzugsweise 6 bis 10 C-Atomen, oder einen Aralkylenrest mit 7 bis 12 C-Atomen, vorzugsweise 8 bis 10 C-Atomen darstellt und R₂, R'₂ unabhängig voneinander für H oder einer der beiden Reste für

   R₁ - NR₃R₄

   stehen, worin
R₁ die gleiche Bedeutung wie vorstehend hat und
R₃ und R₄ unabhängig voneinander für H, (C₁-C₂₀)-Alkyl, vorzugsweise (C₁-C₆)-Alkyl, stehen, wobei diese Alkylreste auch Hydroxygruppen tragen können.

Als Polyamine b1) seien z.B. genannt Methyl-bis-(3-aminopropyl)-amin, Ethyl-bis-(3-aminopropyl)-amin, N-(3-Aminopropyl)-tetramethylendiamin, N,N'-Bis-(3-aminopropyl)-tetramethylendiamin, Polyalkylenpolyamine, wie Dipropylen-(1,2)-triamin, Bis-(3-amino-propyl)-amin, Tripropylen-(1,2)-tetramin und vor allem Diethylentriamin, Triethylentetramin und Tetraethylenpentamin. Polyamine c1) sind beispielsweise: Ethylendiamin, Propylendiamin, 1-Amino-3-methylamino-propan, 2-Methylpentamethylendiamin, Pentamethylendiamin, Hexamethylendiamin, Trimethylhexamethylendiamin, Neopentyldiamin, Octamethylendiamin, Dioxadodecandiamin, cycloaliphatische Diamine wie 1,2-, 1,3- oder 1,4-Cyclohexandiamin, 4,4'-Methylen-bis-cyclohexylamin, Isophorondiamin, Menthandiamin, 4,4'-Diamino-3,3'-dimethyl-di-cyclohexylmethan, 3-Aminomethyl-1-(3-aminopropyl-1-methyl)-4-methylcyclohexan, N-Methylethylendiamin, N-Aminoethylpiperazin, 1,3-Bis-aminomethylbenzol.

Als Alkanolmonoamine sind zum Beispiel solche der Formel

H₂N-R₁-OH (II)

geeignet, in der R₁ die obige Bedeutung hat, wie 2-Aminoethanol, 1-Amino-2-propanol, 3-Amino-1-propanol, 2-Amino-1-butanol, 4-Amino-1-butanol, 5-Amino-1-pentanol, 6-Amino-1-hexanol und deren Isomere, deren Kohlenwasserstoffrest verzweigt ist oder die die Aminogruppe und/oder die Hydroxylgruppe an einem primären oder sekundären C-Atom tragen; ferner solche, die sich von cyclischen Kohlenwasserstoffresten, vorzugsweise mit 5-7 C-Atomen, ableiten.

Die zur Herstellung geeigneter Polyamidoamine mit optimalen Eigenschaften einzuhaltenden Mengenverhältnisse der in Betracht kommenden Komponenten lassen sich durch Vorversuche leicht ermitteln.

Im allgemeinen ist die Mol-Menge an Dicarbonsäure oder deren funktionelle Derivate zu Polyamin/Alkanolmonoamin so, daß das Verhältnis von Carboxylgruppen zu der Summe von primären NH₂-Gruppen 1 : (0,8 - 1,4), vorzugsweise 1 : (0,95 - 1,1) beträgt. Im Falle von Mischungen von Polyaminen und Alkanolmonoaminen liegt das Verhältnis ihrer Molmengen bei 0,6 : 0,4 bis 0,99 : 0,01, vorzugsweise 0,8 : 0,2 bis 0,95 : 0,05.

Die Herstellung der Polyamidoamine kann in üblicher Weise erfolgen, z.B. durch mehrstündiges Erhitzen der entsprechenden Komponenten unter Ausschluß von Sauerstoff auf Temperaturen von 125 bis 250°C, vorzugsweise von 140 bis 180°C, zunächst unter gewöhnlichem Druck und dann unter vermindertem Druck, wobei zur Vermeidung der Dunkelfärbung der Polyamide geringe Mengen an Hydrazinhydrat oder Hydraziden zugesetzt werden können. Die Reaktionszeit hängt von den angewendeten Temperaturen und Drucken ab und liegt im allgemeinen zwischen 4 und 10 Stunden.

Derart hergestellte Polyamidoamine besitzen im allgemeinen ein mittleres Molekulargewicht Mₙ (bestimmt über die Carboxylendgruppen) von mindestens 500, vorzugsweise mindestens 1000 und insbesondere 2000 bis 20000; die Aminzahl liegt zumeist zwischen 200 und 400, vorzugsweise zwischen 250 und 350 mgKOH/g und die Säurezahl zwischen 0 und 50, vorzugsweise 10 und 30 mgKOH/g.

Besonders bevorzugte Polyamidoamine sind solche, die nach ihrer Herstellung mit Epichlorhydrin umgesetzt worden sind. Das Verhältnis der Mol-Menge Epichlorhydrin zur Summe der Äquivalente an freien basischen Aminogruppen im Polyamidoamin beträgt 0,6 : 1 bis 4 : 1, vorzugsweise 1 : 1 bis 1,4 : 1. Unter freien basischen Aminogruppen sind primäre, sekundäre oder tertiäre Aminogruppen zu verstehen. Zum Begriff "Äquivalente" vgl. Pure Appl. Chem. 50 (1978), S. 327-338, bes. S. 337.

Anstelle des Epichlorhydrins kann gegebenenfalls auch das Dichlorhydrin (1,3-Dichlor-2-hydroxypropan) eingesetzt werden, vorzugsweise 1,0 bis 1,4 Mol pro Mol sek. basischer Aminogruppen.

Die Umsetzung mit Epichlorhydrin erfolgt beispielsweise derart, daß man bei einer Konzentration der Reaktanten von etwa 25 bis 50, vorzugsweise 35 bis 45 %, und bei einer Temperatur zwischen 25 und 95°C, vorzugsweise 40 bis 75°C, solange reagieren läßt, bis die Viskosität einer 40%igen Lösung bei 25°C etwa 100 bis 600, vorzugsweise 200 bis 400 mpa•s beträgt.

Ganz besonders bevorzugte Polyamidoamine sind mit Epichlorhydrin umgesetzte Polyamidoamine, die zusätzlich einer Nachbehandlung mit einer anorganischen Base und mit einem wasserlöslichen organischen Mono- oder Polyamin oder Ammoniak oder Gemischen davon unterzogen worden sind. Sinnvollerweise wird anschließend mit Säure auf einen pH-Wert von 1 bis 7 eingestellt.

Die Menge an Base beträgt 0,1 bis 1,0 Mol, vorzugsweise 0,1 bis 0,6 Mol pro Mol eingesetztes Epichlorhydrin. Die Menge an organischen Mono- oder Polyaminen beträgt 0,01 bis 4, vorzugsweise 0,1 bis 1 basische Amin-Äquivalente pro Mol Epichlorhydrin.

Als anorganische Basen können unter anderem eingesetzt werden: Alkalihydroxide, bevorzugt Natrium- und Kaliumhydroxid, Carbonate, Bicarbonate, Erdalkalihydroxide, wie Calciumhydroxid, weiterhin Benzyltrimethylammonium-hydroxid oder entsprechende Gemische.

Als wasserlösliche organische Mono- oder Polyamine für die erfindungsgemäße Nachreaktion kommen vorzugsweise Verbindungen der Formeln III oder IV

NR₁R₂R₃ (III)

oder

R₄R₅N((-R-NR₈)ₙ-R-NR₆R₇ (IV)

infrage, worin
R einen aliphatischen, cycloaliphatischen oder araliphatischen Alkylen- oder Hydroxyalkylenrest mit 1 bis 300 C-Atomen, der durch Heteroatome wie Sauerstoff und Schwefel unterbrochen sein kann, und R₁, R₂, R₃, R₄, R₅, R₆, R₇ unabhängig voneinander Wasserstoff oder einen aliphatischen, cycloaliphatischen oder araliphatischen Alkyl- oder Hydroxyalkylrest mit 1 bis 20 C-Atomen, der durch Heteroatome wie Sauerstoff und Schwefel unterbrochen sein kann, und n eine ganze Zahl von 0 bis 10 bedeuten.

Tertiäre Monoamine der Formel III sind zum Beispiel folgende Verbindungen: Trimethylamin, Triethylamin, Tri-n-propylamin, Triisopropylamin, Tributylamin, N-N-Dimethylethylamin, N,N-Dimethyl-isopropylamin, N,N-Dimethylcyclohexyl-amin, Benzyldimethylamin, Benzyldiethylamin, N-Methylmorpholin, N-Methylpiperidin, Triethanolamin, Dimethylethanolamin, Diethylethanolamin, Diisopropylethanolamin, Dibutylethanolamin, Methyldiethanolamin, Butyl-diethanolamin, Triisopropanolamin, Dimethylisopropanolamin, Diethylisopropanolamin, Dimethylpropanolamin, Diethylpropanolamin, Methyldiisopropanolamin, Dimethyl-neopentanolamin, 6-Dimethylamino-1-hexanol, sekundäre Monoamine sind zum Beispiel: Dimethylamin, Diethylamin, Dipropylamin, Diisopropylamin, Di-n-butylamin, Di-isobutylamin, Di-sek.butylamin (N-(1-Methylpropyl)-2-butanamin, -butanamin, Di-n-Pentyl-amin, Di-hexylamin, Di-2-ethylhexylamin, Methyl-ethylamin, Methyl-propylamin, Methyl-butylamin, Ethyl-isopropylamin, Methyl-iso-butylamin, Ethyl-butylamin, N-n-Propyl-s-butylamin, Di-2-methyl-butylamin, N-Methyl-cyclohexylamin, N-Ethyl-cyclohexylamin, N-Butyl-cyclohexylamin, Dicyclohexylamin, Di-2-methoxyethylamin, Diethanolamin, Di-n-propanolamin, Di-iso-propanolamin, N-Methyl-ethanolamin, N-Ethyl-ethanolamin, N-methyl-isopropanolamin, Cyclohexylethanolamin, Morpholin, Pyrrolidin, primäre Monoamine sind zum Beispiel: Methylamin, Ethylamin, Propylamin, Isopropylamin, n-iso- und sek.Butylamin, t-Butylamin, n-Pentylamin, 3-Amino-pentan, 3-Methyl-1-butanamin, 2-Methyl-2-butanamin, n-Hexylamin, 3-Amino-3-methylpentan, 2-Ethylhexylamin, 3-Methyl-1-butanamin, 3-Methyl-2-butanamin, 1-Methyl-1-butanamin, 2-Ethyl-1-butanamin, 2-Methoxyethylamin, 2-Ethoxyethylamin, 3-Methoxypropylamin, 3-Ethoxypropylamin, 3-(2-Methoxyethoxy)-propyl-amin, Cyclopropanamin, Cyclopentylamin, Cyclohexylamin, 2-Methyl-cyclohexylamin, 1- und 2-Phenylethylamin, Benzylamin, 4-Methoxybenzylamin, 1-Methyl-3-phenylpropylamin, 2-Aminoethanol, 1-Amino-2-propanol, 3-Amino-1-propanol, 2-Amino-1-butanol, 2-Amino-3-pentanol, Polyhydroxymonoamine wie 2-Amino-2-methyl-1,3-propandiol und 2-Amino-2-ethyl-1,3-propandiol und 2-(2-Aminoethoxy)-ethanol).

Polyamine der Formel IV sind zum Beispiel folgende Verbindungen: 1,4-Diazabicyclo(2.2.2)octan, N,N,N', N'-Tetramethylethandiamin, N,N,N',N'-Tetramethyl-1,3-propandiamin, N,N,N',N'-Tetramethyl-1,6-hexandiamin, N,N,N',N'-Tetramethyldiaminodiethylether, N,N,N',N'-Pentamethyldiethylentriamin, Ethylendiamin, N,N'-Dimethyl-1,2-ethandiamin, N-Methyl-1,2-ethandiamin, N-Ethyl-1,2-ethandiamin, N,N-Diethyl-1,2-ethandiamin, N-(2-Hydroxyethyl)1,2-ethandiamin, 1,2-Diaminopropan, 1,3-Diaminopropan, 2-Hydroxy-1,3-diaminopropan, 3-Amino-1-methylaminopropan, N,N-Dimethyl-1,3-propandiamin, N,N'-Dimethyl-1,3-propandiamin, N,N-Diethyl-1,3-propandiamin, N-Cyclohexyl-1,3-propandiamin, 2,2-Dimethyl-1,3-propandiamin, 1,3-Diaminobutan, 1,4-Diaminobutan, 1,3-Bis-(2-hydroxy-ethylamino)propan, 1,4-Bis-methylaminobutan, N,N-Diethyl-1,4-pentandiamin, 2-Methyl-1,5-pentandiamin, 1,5-Pentandiamin, 1,6-Diaminohexan, 2,2,4- und 2,4,4-Trimethyl-6-hexandiamin, 1,2-, 1,3- und 1,4-Diaminocyclohexan, Menthandiamin, Piperazin, 3,5,5-Trimethyl-3-aminomethyl-cyclohexylamin, (Isophorondiamin), 4,4'-Methylen-bis-cyclohexylamin, 4,4'-Methylen-bis-(2-methyl-cyclohexylamin), Bis-(2-aminoethyl)-ether, 2,2'-Bis-methylaminodiethyl-ether, Bis-(3-aminopropyl)-ether, Bis-(3-aminopropyl)-sulfid, 4,9-Dioxadodecan-1,12-diamin, 4,7,10-Trioxatridecan-1,13-diamin, 1,3-Bisaminomethylbenzol (m-Xylylendiamin), TCD-Diamin (Bis(amino-methyl)tricyclodecan), Diethylentriamin, Dipropylentriamin, Tetraethylenpentamin, Pentaethylenhexamin, Tripropylentetramin, höhere Polyalkylenpolyamine, Methyl-bis-(3-aminopropyl)-amin, Dihexamethylentriamin, Aminoethylpiperazin, 3-(2-Aminoethyl)amino-propylamin, N,N'-Bis-(3-aminopropyl)ethylendiamin, Ethyl-bis-(3-aminopropyl)amin, 2-Hydroxyethyl-bis-(3-aminopropyl)-amin, N-(3-Aminopropyl)-tetramethylendiamin, N,N'-Bis-(3-aminopropyl)-tetramethylendiamin, 1,3-Bis-(2-aminoethylamino)propan, 3-(3-Diethyl-amino-propylamino)-propylamin, 2,2'-Bis-(2-amino-ethylamino)-diethylether, 1,6-Bis-(2-aminoethylamino)-hexan, 1,6-Bis-(3-amino-propylamino)-hexan, Bis-(6-amino-n-hexyl)amin, Polyalkylenpolyamine, Polyethylenimine, Polyamidoamine oder Gemische hieraus. Als Polyamidoamine werden hierbei die gleichen Polyamidoamine genommen, wie zuvor erwähnt und beschrieben.

Die Nachbehandlung erfolgt beispielsweise dadurch, daß die nach der Umsetzung mit Epichlorhydrin erhaltene Lösung mit einer wäßrigen Lösung einer anorganischen Base und mit einem wasserlöslichen organischen Mono- oder Polyamin oder Ammoniak oder Gemischen davon bei einer Konzentration der Reaktionsteilnehmer von 10 bis 50, vorzugsweise 15 bis 30% und einer Temperatur zwischen 25 und 95°C, vorzugsweise 40 bis 75°C, umgesetzt wird, bis die Viskosität einer 12,5%igen Lösung bei 25°C 10 bis 200 mPa•s, vorzugsweise 15 bis 80 mPa•s beträgt. Anschließend wird der pH-Wert durch Säurezugabe auf einen pH-Wert von 1 bis 7, vorzugsweise 1,5 bis 5, eingestellt.

Die Zugabe des Mono- oder Polyamins kann unmittelbar nach Zugabe der anorganischen Base bis kurz vor Erreichen der gewünschten Viskosität bzw. kurz vor der Säurezugabe erfolgen. Besonders bevorzugt ist die Zugabe des Mono- oder Polyamins in der Mitte dieses Reaktionszeitraumes.

In besonderen Fällen kann die Zugabe des Mono- oder Polyamins auch vor der Basenzugabe erfolgen. In diesem Fall muß aber die Anlagerung des Epichlorhydrins an das Polyamidoamin bereits abgeschlossen sein.

Als halogenfreie Säuren kommen vor allem Schwefel-, Phosphor- oder Essigsäure in Betracht, deren Anhydride oder auch saure Salze dieser Säuren.

Die ganz besonders bevorzugten Polyamidoamine, die wie oben beschrieben mit Epichlorhydrin umgesetzt und einer Nachbehandlung unterzogen worden sind, besitzen im allgemeinen ein mittleres Molekulargewicht Mₙ von mindestens 500, vorzugsweise von mindestens 1000 und insbesondere von 1000 bis 300000. Der Gehalt an organisch gebundenem Chlor liegt im allgeminen zwischen 0,01 und 2,5, vorzugsweise zwischen 0,01 und 2 und insbesondere zwischen 0,01 und 1 Gew.-%. In einer 12,5%igen Lösung übersteigt dieser Chlorgehalt nicht Werte von 0,3 Gew.-% und liegt im allgemeinen zwischen 0,001 und 0,3, vorzugsweise zwischen 0,001 und 0,25 und insbesondere zwischen 0,001 und 0,125 Gew.-% bezogen auf die Lösung.

Die den erfindungsgemäßen Hydrogelen zugrunde liegenden Polymere aus (co)polymerisierten hydrophilen Monomeren bzw. Pfropf(co)polymeren sind bekannt und beispielsweise in den oben zitierten Literaturstellen beschrieben.

Bevorzugt sind Polymere aus hydrophilen Monomeren wie beispielsweise Acrylsäure, Methacrylsäure, deren (C₁-C₆)-Alkyl-, Polyoxy(C₁-C₃)alkyl- und Hydroxy(C₁-C₆)-alkylester, Crotonsäure, 2-Acrylamido-2-methyl-propansulfonsäure und -phosphonsäure, Vinylphosphonsäure und deren Ester, Vinylamide, Vinylester und (C₂-C₆)-Alkenylsulfonsäuren oder Gemischen davon.

Bevorzugt sind auch Pfropf(co)polymere der genannten Monomeren, besonders bevorzugt von Acrylsäure und Methacrylsäure, wobei als Pfropfgrundlagen Stärke, Cellulose, Cellulosederivate, Alginate oder andere Biopolymere, hydrophile Polyester, Polyalkylensulfonsäuren, Polyvinylalkohol oder Polyalkylenoxide dienen können.

Geeignete Polyalkylenoxide haben beispielsweise die Formel 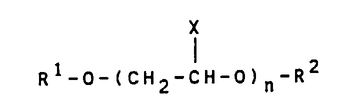
worin R¹ und R² unabhängig voneinander Wasserstoff, Alkyl, Alkenyl oder Aryl,
X Wasserstoff oder Methyl und
n eine ganze Zahl von 1 bis 10000 bedeuten.

R¹ und R² bedeuten bevorzugt Wasserstoff, (C₁-C₆)-Alkyl, (C₂-C₆)-Alkenyl oder Phenyl.
Bevorzugt sind daneben auch die in US 4,931,497 und US 5,011,892 beschriebenen Pfropfpolymere.

Die den erfindungsgemäßen Hydrogelen zugrunde liegenden Polymere bzw. Pfropfpolymere können auch bereits vorvernetzt sein, wobei geeignete Vernetzer beispielsweise Methylenbisacrylamid, Bisacrylamidoessigsäure, Alkenylphosphon- oder -phosphinsäureestern, Trimethylolpropantri(meth)acrylat oder Tetraallyloxyethan sind.

Die erfindungsgemäßen Hydrogele werden durch Umsetzung von Polymeren aus (co)polymerisierten hydrophilen Monomeren bzw. von Pfropf(co)polymeren mit Polyamidoaminen in wäßrigem Medium erhalten. Es ist dabei bevorzugt, die Polymeren in Form wäßriger Gele einzusetzen, die vorteilhafterweise vor der Umsetzung in geeigneten Vorrichtungen zerkleinert werden. Die Umsetzung mit dem Polyamidoamin erfolgt in diesem Fall beispielsweise in einem Kneter, wobei eine zusätzliche Wasserzugabe nicht erforderlich ist.

Vorteilhafterweise wird die Umsetzung bei Temperaturen über 80°C ausgeführt. Bevorzugt sind Temperaturen von 80 bis 120°C.

Die Polyamidoamine werden vorteilhafterweise in solchen Mengen eingesetzt, daß bis zu 80 Mol% der im Polymer enthaltenen Carboxylgruppen neutralisiert werden. Bevorzugt werden die Polyamidoamine in Mengen von 0,2 bis 1,0 Gew% bezogen auf die Ausgangspolymere (gerechnet als 100%ige Ware) eingesetzt.

Die erfindungsgemäßen hydrophilen, hochquellfähigen Hydrogele sind als Absorptionsmittel für Wasser, wässrige Lösungen und wässrige Körperflüssigkeiten in hervorragender Weise geeignet und zeichnen sich durch ein hohes Aufnahmevermögen, insbesondere auch unter Druckbelastung, auf. Sie können deshalb insbesondere bei der Herstellung von Hygieneartikeln wie Baby- und Erwachsenenwindeln, Binden, Tampons und anderen saugfähigen Produkten Verwendung finden.

Die erfindungsgemäßen hydrophilen, hochquellfähigen Hydrogele können auch zur Herstellung von Flächen- und Körpergebilden mit homogen Wasser-absorbierenden Eigenschaften verwendet werden. Dazu werden die genannten Gebilde beispielsweise mit einer wäßrigen Lösung aus Hydrogel und Polyamidoamin durch Aufsprühen imprägniert und bis Abschluß der Vernetzung bei Raumtemperatur oder erhöhten Temperaturen gelagert. Geeignete Flächen- und Körpergebilde können aus Naturfasern und/oder Synthesefasern bestehen. Bevorzugte Gebilde sind Saugkissen aus Cellulose-Fluff und Mischungen aus Cellulose-Fluff und Zellwolle und/oder Polyester.

Durch diese Verwendung der erfindungsgemäßen Hydrogele als Binder/Verfestiger kann sowohl Festigkeit, als auch Saugkapazität der behandelten Gebilde erheblich verbessert werden. Konventionelle Polymere mit absorbierenden Eigenschaften sind in der Regel zur Herstellung der genannten Gebilde nicht geeignet.

### Beispiel 1:

In einem durch geschäumtes Kunststoffmaterial gut isolierten Polyethylengefäß mit einem Fassungsvermögen von 10 1 werden 4780 g E-Wasser vorgelegt, 1233 g Natriumbicarbonat darin suspendiert und langsam 1994 g Acrylsäure so zudosiert, daß ein Überschäumen der Reaktionslösung vermieden wird, wobei sich diese auf eine Temperatur von ca. 5-3°C abkühlt. Bei einer Temperatur von 4°C werden die Initiatoren, ein Redoxsystem, bestehend aus 2,2 g 2,2'-Azobis-amidino-propan-dihydrochlorid, gelöst in 20 g E-Wasser, 4 g Kaliumperoxodisulfat, gelöst in 150 g E-Wasser, sowie 0,4 g Ascorbinsäure, gelöst in 20 g E-Wasser nacheinander zugegeben und gut verrührt. Die Reaktionslösung wird daraufhin ohne Rühren stehen gelassen, wobei durch einsetzende Polymerisation, in dessen Verlauf die Temperatur bis auf ca. 85°C ansteigt, ein hochviskoses, leicht fädenziehendes Gel entsteht. Dieses wird anschließend in einen Kneter überführt, mit 2-Gew.% (bezogen auf Acrylsäure) Festsubstanz eines handelsüblichen kationischen Polyamidoaminharzes (KYMENE 557H® der Hercules Corp., USA) versetzt, homogen verknetet, zerkleinert, im Luftstrom bei 180°C getrocknet, gemahlen und gesiebt. Man erhält ein wasserquellbares Produkt mit einem Wasserabsorptionsvermögen selbst unter Druck von 40 g/cm² vom Mehrfachen des Eigengewichtes.

### Beispiel 2:

In gleicher Vorgehensweise wie in Beispiel 1 beschrieben, wird ein wasserlösliches Polymergel hergestellt, das jetzt mit 2,5-Gew.% (bezogen auf Acrylsäure) Wirksubstanz eines Polyamidoamins aus Adipinsäure, Ethylendiamin, Ethanolamin und Ethylentriamin nach homogener Durchmischung in einem Kneter zur Reaktion gebracht wird. Man erhält nach Trocknung bei 180°C ein nicht mehr wasserlösliches, sondern nur noch quellbares Produkt mit einem Wasserretentionsvermögen selbst unter Ausübung eines Druckes von 60 g/cm² vom Mehrfachen des Eigengewichtes.

### Beispiel 3:

In einem durch geschäumtes Kunststoffmaterial gut isolierten Polyethylengefäß mit einem Fassungsvermögen von 10 1 werden 5200 g E-Wasser/Eis vorgelegt, 695 g Natriumcarbonat darin suspendiert und langsam 1986 g Acrylsäure so zudosiert, daß ein Überschäumen der Reaktionslösung vermieden wird, wobei sich diese auf eine Temperatur von ca. 5-3°C abkühlt. Es werden 25 g eines als Pfropfmatrix dienenden Umsetzungsproduktes aus 2 Mol Maleinsäureanhydrid und 1 Mol eines EO/PyO(60/40)-Blockpolymerisates zugegeben sowie 4 g Trimethylolpropantriacrylat und 5 g eines Natriumdiisooctylsulfosuccinat (REWOPOL V 2133 der Fa. Rewo, Steinau). Bei einer Temperatur von 4°C werden die Initiatoren, ein Redoxsystem, bestehend aus 2,2 g 2,2'-Azobis-amidinopropan-dihydrochlorid, gelöst in 20 g E-Wasser, 4 g Kaliumperoxodisulfat, gelöst in 150 g E-Wasser, sowie 0,4 g Ascorbinsäure, gelöst in 20 g E-Wasser nacheinander zugegeben und gut verrührt. Die Reaktionslösung wird daraufhin ohne Rühren stehen gelassen, wobei durch einsetzende Polymerisation, in dessen Verlauf die Temperatur bis auf ca. 85°C ansteigt, ein festes Gel entsteht, das anschließend mechanisch zerkleinert wird. 3000 g des zerkleinerten Gels werden mit 370 g Natronlauge 50%ig (Neutralisationsgrad der Acrylsäure = 73 Mol-%) versetzt, zweimal durchgeknetet, mit 154 g (0,75-Gew.% bezogen auf Acrylsäure) eines auf 5% verdünnten und auf pH 6,0 eingestellten, nicht quarternisierten Polyamidoaminproduktes, hergestellt gemäß Beispiel 6 der EP 0 349 935, versetzt, wiederum zweimal durchgeknetet, anschließend bei Temperaturen über 150°C in dünner Schicht getrocknet, gemahlen und gesiebt. Man erhält ein Produkt, im Wesentlichen gekennzeichnet durch folgende physikalische Daten, alle gemessen in NaCl 0,9% : Extrahierbare Anteile (lh-Wert) = 7,8%, Absorption unter Druck (AUL) (20 g/cm²) = 18,7 g/g, Free Swell Capacity (20 Min.) = 55 g/g.
Zum Vergleich:
Wird oben beschriebenes Polymer vor dem Trocknen nicht mit dem genannten Polyamidoaminharz versetzt, so weist das dann unter gleichen Bedingungen aufgearbeitete Produkt folgende Werte auf:
Extractables = 9,9%, AUL = 9,6 g/g, FSC = 58 g/g.

### Beispiel 4:

In einem durch geschäumtes Kunststoffmaterial gut isolierten Polyethylengefäß mit einem Fassungsvermögen von 10 1 werden 4950 g E-Wasser/Eis vorgelegt, 553 g Natriumbicarbonat darin suspendiert und langsam 1986 g Acrylsäure so zudosiert, daß ein Überschäumen der Reaktionslösung vermieden wird, wobei sich diese auf eine Temperatur von ca. 5-3°C abkühlt. Es werden 1,5 g eines Polyglykol-300-di-Maleinsäurehalbesters zugegeben sowie 4,7 g Tetraallyloxyethan, 2,7 g Methylenbisacrylamid, 3,9 g Sorbitanmonolaurat und 22 g Harnstoff. Bei einer Temperatur von 4°C werden die Initiatoren, ein Redoxsystem, bestehend aus 1,7 g 2,2'-Azobis-amidinopropan-dihydrochlorid, gelöst in 20 g E-Wasser, 3,9 g Kaliumperoxodisulfat, gelöst in 150 g E-Wasser, sowie 0,33 g Ascorbinsäure, gelöst in 20 g E-Wasser nacheinander zugegeben und gut verrührt. Die Reaktionslösung wird daraufhin ohne Rühren stehen gelassen, wobei durch einsetzende Polymerisation, in dessen Verlauf die Temperatur bis auf ca. 85°C ansteigt, ein festes Gel entsteht, das anschließend mechanisch zerkleinert wird, mit 970 g Natronlauge 50%ig (Neutralisationsgrad der Acrylsäure = 73 Mol-%) versetzt, zweimal durchgeknetet, mit 212 g eines auf 5% verdünnten handelsüblichen Polyamidoamins auf Basis Adipinsäure, Diethylentriamin, Ethylendiamin und Ethanolamin, das mit Epichlorhydrin umgesetzt und einer Alkali-Amin-Behandlung unterzogen wurde, versetzt, wiederum zweimal durchgeknetet, anschließend bei Temperaturen über 150°C im Luftstrom getrocknet, gemahlen und gesiebt. Man erhält ein Produkt, im Wesentlichen gekennzeichnet durch folgende physikalische Daten, alle gemessen in NaCl 0,9% : Extrahierbare Anteile (1h-Wert) = - 3,7%, Absorption unter Druck (20 g/cm²) = 28 g/g, Free Swell Capacity (20 Min.) = 45 g/g.
Zum Vergleich:
Wird oben beschriebenes Polymer vor dem Trocknen nicht mit dem erfindungsgemäßen Polyamidoaminharz versetzt, so weist das dann unter gleichen Bedingungen aufgearbeitete Produkt folgende Werte auf:
Extractables = 7,9%, AUL = 14,5 g/g, FSC = 543 g/g.

### Beispiel 5:

Unter adiabatischen Bedingungen werden in einem 5 1 zylindrischen Weithalsreaktionskolben 3650 g E-Wasser vorgelegt, 500 g einer frisch aufgekochten Stärkelösung aus 50 g Maisstärke und 450 g E-Wasser, 850 g Acrylsäure sowie 4,0 g Tetraallyloxyethan darin gelöst und auf 20°C eingestellt. Es wird Stickstoff in die Monomerlösung eingeleitet (ca. 21/Min.), um den Sauerstoffgehalt zu erniedrigen. Bei einem Gehalt von ca. 0,8 ppm O₂ werden 64 g einer 4%igen wässrigen Lösung von 2,2'-Azobis(amidinopropan)dihydrochlorid zugegeben, nach weiterem N₂-Einleiten und einem O₂-Gehalt von 0,08 ppm werden 11 g einer 0,75%igen H₂O₂-Lösung zugegeben und schließlich bei einem O₂-Gehalt von ca. 0,01 ppm werden 10,5 g einer 0,15%igen Ascorbinsäurelösung zugegeben. Durch einsetzende Polymerisation, in deren Verlauf die Temperatur bis auf ca. 90°C ansteigt, entsteht ein festes Gel, das anschließend mechanisch zerkleinert, mit 1225 g Natronlauge 27%ig versetzt (Neutralisationsgrad der Acrylsäure = 70 Mol-%), zweimal durchgeknetet, mit 127,5 g des auf 5% verdünnten Polyamidoamins gemäß Beispiel 4 versetzt, wiederum zweimal durchgeknetet und anschließend bei Temperaturen über 100°C in dünner Schicht getrocknet, gemahlen und gegebenenfalls gesiebt. Man erhält ein Produkt, im Wesentlichen gekennzeichnet durch folgende physikalische Daten, alle gemessen in NaCl 0,9% : Extrahierbare Anteile (1h-Wert) = 1,7%, Absorption unter Druck (20 g/cm²) = 31 g/g, Free Swell Capacity (20 Min.) = 49 g/g.
Zum Vergleich:
Wird oben beschriebenes Polymer vor dem Trocknen nicht mit dem erfindungsgemäßen Polyamidoaminharz versetzt, so weist das dann unter gleichen Bedingungen aufgearbeitete Produkt folgende Werte auf:
Extractables = 6,9%, AUL = 12,5 g/g, FSC = 58 g/g.

### Beispiel 6:

Eine 10%ige wässrige Copolymerenlösung mit der Monomerenzusammensetzung von 90 Gew.-% Acrylsäure und 10 Gew.-% Acrylsäure und 10 Gew.-% Vinylphosphonsäure, mit NaOH teilneutralisiert auf einen pH-Wert von 5,5 - 6,0, einem K-Wert nach Fikentscher von 187,5 und versetzt mit 1,0 Gew.-% an Wirksubstanz (bezogen auf Polyacrylsäure) eines handelsüblichen Polyamidoamins auf Basis Adipinsäure, Diethylentriamin, Ethylendiamin und Ethanolamin, das mit Epichlorhydrin umgesetzt ist, wurde gleichmäßig beidseitig auf ein Saugkissen aus Cellulose-Fluff (ca. 6x20x1,5 cm / BxLxH,) so aufgesprüht, daß das Saugkissen mit 1% des Polymerfeststoffes, bezogen auf das Trockengewicht des Saugkissens, beaufschlagt wird. Nach 24 stündiger Lagerung bei Raumtemperatur an der Luft oder entsprechender kürzerer Verweilzeit bei höheren Temperaturen wurden die so behandelten gegen entsprechende unbehandelte Saugkissen auf Festigkeit und Saugkapazität untersucht. Die Festigkeit wurde derart geprüft, daß die Saugkissen in einem speziellen Zerwirbelungsgefäß einem definierten Luftstrom ausgesetzt waren. Die durch die Wirbelung entstandenen, vom Saugkissen abgetrennten Bestandteile wurden über ein Sieb definierter Maschenweite abgesaugt. Bestimmt wurde anschließend der Anteil an unzerstörtem, vor dem Sieb zurückgebliebenem nicht abgesaugtem Kissenmaterial, in % bezogen auf Ausgangsgewicht.

Die Saugkapazität wurde folgendermaßen bestimmt:
Das Saugkissen wurde plan auf einem Siebblech liegend eine Minute in 0,9%ige NaCl-Lösung getaucht. Anschließend wurde das Siebblech herausgeholt und eine Minute abtropfen lassen. Hierzu wurde die Versuchsanordnung etwa 45° geneigt. Es wurde die Gewichtszunahme pro Gramm Saugkissen berechnet.
In Bezug auf Festigkeit konnte bei den behandelten Saugkissen im Vergleich zu unbehandelten eine Verbesserung um ca. 20%, in Bezug auf Saugkapazität um ca. 10% festgestellt werden.

### Beispiel 7:

Eine 10%ige wässrige Lösung eines in an sich bekannter Weise hergestellten Acrylsäurehomopolymers, mit NaOH zu 70 Mol-% neutralisiert und einem K-Wert nach Fikentscher von 207, mit 1,5 Gew.-% (bezogen auf Acrylsäure) Wirksubstanz eines Kondensationsproduktes aus 10,43 Mol Adipinsäure, 6,78 Mol Diethylentriamin, 3,13 Mol Ethylendiamin und 1,05 Mol 2-Aminoethanol, hergestellt gemäß Beispiel 3 der DE 4114657 versetzt und diese Lösung nach Verdünnung in eine sprühfähige Form vollkommen analog zu Beispiel 6 als Verfestiger/Binder von Vlies strukturen mit verbessertem Saugvermögen eingesetzt.
In Bezug auf Festigkeit konnte bei den behandelten Saugkissen im Vergleich zu unbehandelten eine Verbesserung um ca. 10%, in Bezug auf Saugkapazität um ca. 5% festgestellt werden.

## Patentansprüche

1. Hydrophiles, hochquellfähiges Hydrogel auf Basis vorvernetzter (co)polymerisierter hydrophiler Monomerer oder auf Basis vorvernetzter Pfropf(co)polymerer, dadurch gekennzeichnet, daß es mit einem Polyamidoamin in wäßrigem Medium nachvernetzt ist.

2. Hydrogel gemäß Anspruch 1, dadurch gekennzeichnet, daß als Polyamidoamine Umsetzungsprodukte von
a) gesättigten oder olefinisch-ungesättigten aliphatischen C₃-C₁₀-Dicarbonsäuren oder deren funktionellen Derivaten bzw. mindestens 3 C-Atome enthaltenden ω-Aminocarbonsäuren oder deren Lactamen mit
b1) aliphatischen Polyaminen, die mindestens zwei primäre und mindestens eine weitere, und zwar sekundäre und/oder tertiäre Aminogruppe enthalten, oder
b2) einem Gemisch der Polyamine b1) mit
c1) Polyaminen, die mindestens eine primäre und gegebenenfalls mindestens eine sekundäre Aminogruppe enthalten und nicht unter die Definition b1) fallen, wobei diese Polyamine in solchen Mengen verwendet werden können, daß deren Anteil an primären Aminogruppen, bezogen auf die Gesamtmenge der primären Aminogruppen, 70 %, nicht übersteigt, und/oder gegebenenfalls
c2) Alkanolmonoaminen mit 2 bis 20 C-Atomen und 1 bis 3 OH-Gruppen
eingesetzt werden.

3. Hydrogel gemäß Anspruch 1 und/oder 2, dadurch gekennzeichnet, daß als Polyamidoamine Umsetzungsprodukte von Bernsteinsäure, Glutarsäure oder Adipinsäure mit Ethylendiamin und/oder Diethylentriamin und/oder Triethylentetramin und/oder Tetraethylenpentamin und/oder 2-Aminoethanol eingesetzt werden.

4. Hydrogel gemäß einem oder mehreren der Ansprüche 1 bis 3, dadurch gekennzeichnet, daß mit Epichlorhydrin umgesetzte Polyamidoamine eingesetzt werden.

5. Hydrogel gemäß einem oder mehreren der Ansprüche 1 bis 4, dadurch gekennzeichnet, daß mit Epichlorhydrin umgesetzte und mit einer anorganischen Base und mit einem wasserlöslichen organischen Mono- oder Polyamin oder Ammoniak oder Gemischen davon nachbehandelte Polyamidoamine eingesetzt werden.

6. Hydrogel gemäß einem oder mehreren der Ansprüche 1 bis 5, dadurch gekennzeichnet, daß als (co)polymerisierte hydrophile Monomere Polymere von Acrylsäure, Methacrylsäure, deren (C₁-C₆)-Alkyl-, Polyoxy(C₁-C₃)-alkyl- und Hydroxy(C₁-C₆)alkylester, Crotonsäure, 2-Acrylamido-2-methylpropansulfonsäure und -phosphonsäure, Vinylphosphonsäure und deren Ester, Vinylamide, Vinylester und (C₂-C₆)-Alkenylsulfonsäuren oder Gemischen davon und als Pfropf(co)polymere Pfropf(co)polymere der genannten Monomere, wobei als Pfropfgrundlagen Stärke, Cellulose, Cellulosederivate, Alginate oder andere Biopolymere, hydrophile Polyester, Polyalkylensulfonsäuren, Polyvinylalkohol oder Polyalkylenoxide dienen, eingesetzt werden.

7. Verwendung eines Hydrogels gemäß einem oder mehreren der Ansprüche 1 bis 7 als Absorptionsmittel für Wasser, wässrige Lösungen und wässrige Körperflüssigkeiten.

8. Verwendung eines Hydrogels gemäß einem oder mehreren der Ansprüche 1 bis 7 zur Herstellung von Flächen- und Körpergebilden mit homogen Wasser-absorbierenden Eigenschaften.

9. Flächen- und Körpergebilde mit homogen Wasser-absorbierenden Eigenschaften, dadurch gekennzeichnet, daß sie ein Hydrogel gemäß einem oder mehreren der Ansprüche 1 bis 7 enthalten.

## Claims

1. Hydrophilic highly swellable hydrogel based on precrosslinked (co)polymerized hydrophilic monomers or based on precrosslinked graft (co)polymers, characterized in that it is post-crosslinked with a polyamidoamine in an aqueous medium.

2. Hydrogel according to Claim 1, characterized in that reaction products of
a) saturated or olefinically unsaturated aliphatic C₃-C₁₀-dicarboxylic acids or functional derivatives thereof or ω-aminocarboxylic acids containing at least 3 C atoms or lactams thereof with
b1) aliphatic polyamines which contain at least two primary and at least one further, secondary and/or tertiary amino group, or
b2) a mixture of polyamines b1) with
c1) polyamines which contain at least one primary and if appropriate at least one secondary amino group and do not fall under the definition b1), it being possible for these polyamines to be used in amounts such that their content of primary amino groups, based on the total amount of primary amino groups, does not exceed 70%, and/or, if appropriate,
c2) alkanolmonoamines having 2 to 20 C atoms and 1 to 3 OH groups
are employed as the polyamidoamines.

3. Hydrogel according to Claim 1 and/or 2, characterized in that reaction products of succinic acid, glutaric acid or adipic acid with ethylenediamine and/or diethylenetriamine and/or triethylenetetramine and/or tetraethylenepentamine and/or 2-aminoethanol are employed as the polyamidoamines.

4. Hydrogel according to one or more of Claims 1 to 3, characterized in that polyamidoamines which have been reacted with epichlorohydrin are employed.

5. Hydrogel according to one or more of Claims 1 to 4, characterized in that polyamidoamines which have been reacted with epichlorohydrin and after-treated with an inorganic base and with a water-soluble organic mono- or polyamine or ammonia or mixtures thereof are employed.

6. Hydrogel according to one or more of Claims 1 to 5, characterized in that polymers of acrylic acid, methacrylic acid, (C₁-C₆)-alkyl, polyoxy(C₁-C₃)-alkyl and hydroxy(C₁-C₆)-alkyl esters thereof, crotonic acid, 2-acrylamido-2-methylpropanesulphonic acid and -phosphonic acid, vinylphosphonic acid and esters thereof, vinylamides, vinyl esters and (C₂-C₆)-alkenylsulphonic acids or mixtures thereof are employed as the (co)polymerized hydrophilic monomers and graft (co)polymers of the monomers mentioned are employed as the graft (co)polymers, starch, cellulose, cellulose derivatives, alginates or other biopolymers, hydrophilic polyesters, polyalkylenesulphonic acids, polyvinyl alcohol or polyalkylene oxides being used as the grafting bases.

7. Use of a hydrogel according to one or more of Claims 1 to 6 as an absorbent for water, aqueous solutions and aqueous body fluids.

8. Use of a hydrogel according to one or more of Claims 1 to 6 for the preparation of flat and bodied structures having homogeneously water-absorbing properties.

9. Flat and bodied structures having homogeneously water-absorbing properties, characterized in that they comprise a hydrogel according to one or more of Claims 1 to 6.

## Revendications

1. Hydrogel à forte capacité de gonflement hydrophile, à base de monomères hydrophiles (co)polymérisés préréticulés ou à base de (co)polymères greffés préréticulés, caractérisé en ce qu'il subit une post-réticulation avec une polyamidoamine en milieu aqueux.

2. Hydrogel selon la revendication 1, caractérisé en ce que l'on utilise comme polyamidoamines des produits de réaction
a) d'acides dicarboxyliques en C₃-C₁₀ aliphatiques saturés ou à insaturation oléfinique, ou de leurs dérivés fonctionnels, respectivement d'acides ω-aminocarboxyliques contenant au moins 3 atomes de carbone ou leurs lactames avec
b₁) des polyamines aliphatiques, qui contiennent au moins deux groupes amino primaires et au moins un autre groupe amino, c'est-à-dire secondaire et/ou tertiaire, ou
b₂) un mélange des polyamines b₁) avec
c₁) des polyamines, qui contiennent au moins un groupe amino primaire et éventuellement au moins un groupe amino secondaire et qui n'entrent pas dans le cadre de la définition b₁), ces polyamines pouvant être utilisées dans de telles quantités, que leur taux en groupes amino primaires, par rapport à la quantité totale des groupes amino primaires, ne soit pas supérieur à 70 %, et/ou éventuellement
c₂) des alcanolmonoamines avec 2 à 20 atomes de carbone et 1 à 3 groupes OH.

3. Hydrogel selon la revendication 1 et/ou 2, caractérisé en ce que l'on utilise en tant que polyamidoamines des produits de réaction de l'acide succinique, de l'acide glutarique ou de l'acide adipique avec l'éthylènediamine et/ou la diéthylènetriamine et/ou la diéthylènetétramine et/ou la tétraéthylènepentamine et/ou le 2-aminoéthanol.

4. Hydrogel selon une ou plusieurs des revendications 1 à 3, caractérisé en ce que l'on utilise des polyamidoamines ayant réagi avec l'épichlorhydrine.

5. Hydrogel selon une ou plusieurs des revendications 1 à 4, caractérisé en ce que l'on utilise des polyamidoamines ayant réagi avec l'épichlorhydrine et ayant subi un post-traitement avec une base organique et une mono- ou polyamine organique hydrosoluble ou l'ammoniac ou leurs mélanges.

6. Hydrogel selon une ou plusieurs des revendications 1 à 5, caractérisé en ce que l'on utilise comme polymères des monomères (co)polymérisés hydrophiles d'acide acrylique, d'acide méthacrylique, de leurs esters d'alkyles en C₁-C₆, de polyoxy(alkyles en C₁-C₃) et d'hydroxy(alkyles en C₁-C₆), l'acide crotonique, l'acide 2-acrylamido-2-méthylpropanesulfonique et -phosphonique, l'acide vinylphosphonique et ses esters, des vinylamides, des esters vinyliques et des acides (alcényle en C₂-C₆)sulfoniques ou leurs mélanges, et des (co)polymères greffés des monomères précités, en utilisant en tant que bases de greffage l'amidon, la cellulose, des dérivés de cellulose, des alginates ou autres biopolymères, des polyesters hydrophiles, des acides polyalkylène-sulfoniques, du poly(alcool vinylique) ou des poly(oxyalkylènes).

7. Utilisation d'un hydrogel selon une ou plusieurs des revendications 1 à 6, en tant qu'agent absorbant d'eau, de solutions aqueuses et de fluides corporels aqueux.

8. Utilisation d'un hydrogel selon une ou plusieurs des revendications 1 à 7 pour la préparation d'objets plats et tridimensionnels ayant des caractéristiques homogènes d'absorption d'eau.

9. Objets plats et tridimensionnels ayant des caractéristiques homogènes d'absorption d'eau caractérisé en ce qu'ils contiennent un hydrogel selon une ou plusieurs des revendications 1 à 7.
